# EUROPÄISCHE PATENTSCHRIFT

(11) **EP 3 302 662 B1**
(45) Veröffentlichungstag und Bekanntmachung des Hinweises auf die Patenterteilung: **18.09.2019**
(21) Anmeldenummer: 16724884.8
(22) Anmeldetag: 23.05.2016
(51) Int. Cl.: A61M 16/00, A61M 16/20, A61M 16/04, A61M 16/08

(54) **ANORDNUNG MIT GASSTROMUMKEHRELEMENT UND EINEM SCHALTBAREN ANSCHLUSS**
ARRANGEMENT HAVING A GAS FLOW REVERSING ELEMENT AND A SWITCHABLE CONNECTION
AGENCEMENT PRÉSENTANT UN ÉLÉMENT D'INVERSION DE FLUX DE GAZ ET RACCORD COMMUTABLE

(30) Priorität: 01.06.2015 DE 102015108593
(43) Veröffentlichungstag der Anmeldung: 11.04.2018
(73) Patentinhaber: Ventinova Technologies B.V., 5612 AR Eindhoven (NL)
(72) Erfinder: ENK, Dietmar, 48653 Coesfeld (DE)
(74) Vertreter: Rössler, Matthias
(86) Internationale Anmeldenummer: PCT/EP2016/061558
(87) Internationale Veröffentlichungsnummer: WO 2016/193035

(56) Entgegenhaltungen:
- EP-A2- 0 130 930
- WO-A1-2015/004229
- WO-A2-2008/113752
- DE-A1-102009 029 959
- US-A- 5 669 380

## Beschreibung

Der Gegenstand der vorliegenden Erfindung bezieht sich auf eine Anordnung, zumindest aufweisend einen schaltbaren Anschluss und ein Gasstromumkehrelement. Eine Anwendung ist der Anschluss einer solchen Anordnung an einen Gasvorrat einer Druckgasquelle einerseits und an einen in den Atemweg eines Patienten einbringbaren Katheter oder an eine Kanüle (im Folgenden allgemein als Lumen bezeichnet).

Bei der Beatmung eines Patienten wird normalerweise eine Maske oder ein Tubus eingesetzt, über die bzw. den dem nach außen abgedichteten Atemweg mit niedrigem Druck ein Gas oder Gasgemisch, insbesondere Sauerstoff und Luft, zugeführt wird. Alternativ kann man ein solches Gas oder Gasgemisch aber auch mit hohem Druck und hohem Fluss stoßweise durch einen dünnen, ungeblockten Katheter in den nach außen offenen Atemweg injizieren (sogenannte Jet-Ventilation). Dieses Verfahren wird heute insbesondere bei diagnostischen und therapeutischen Eingriffen im Bereich des oberen Atemweges eingesetzt (endotracheale oder transtracheale Jet-Ventilation). Dabei kann dieses Verfahren in Notfallsituationen auch außerhalb von Krankenhäusern oder in stationären Situationen in Krankenhäusern angewendet werden.

Bei der transtrachealen Jet-Ventilation kann ein Patient durch einen durch die Haut direkt in die Luftröhre eingebrachten Katheter bzw. eine so platzierte Kanüle mit Sauerstoff versorgt werden. Diese Verfahren (trans-/ endotracheal) sind Bestandteil der heute gültigen Algorithmen zum Management eines schwierigen Atemweges und insbesondere der Situation, in der ein Patient konventionell nicht zu beatmen bzw. zu intubieren ist (sogenannte "cannot ventilate, cannot intubate"-Situation).

Unter ungünstigen Bedingungen kann aber gerade Jet-Ventilation auch potentiell lebensgefährlich sein. Ist der Atemweg eines Patienten weitgehend oder sogar vollständig z.B. durch Schwellung oder Blutung verlegt, wird die Lunge des Patienten durch die Injektion von Atemluft mehr und mehr gebläht. Dabei droht eine Lungenzerreißung (sogenanntes Barotrauma). Weiterhin kann die Drucksteigerung im Brustkorb zu einer für den Patienten nicht minder gefährlichen Kreislaufstörung bzw. zu einem Kreislaufzusammenbruch führen, da das Blut nicht mehr in ausreichendem Maße zum Herzen zurückfließen kann.

Aus der WO 2008/113752 A1 ist ein Gasstromumkehrelement bekannt, das in der hier beanspruchten Anordnung verwendet werden kann.

Aus der US 61/844746 ist ein weiteres Gasstromumkehrelement mit einer Bypassleitung bekannt, das ebenfalls in der hier vorgeschlagenen Anordnung verwendet werden kann.

Die DE 10 2009 029 959 A1 offenbart ein Beatmungsgerät, insbesondere zur notfallmäßigen transtrachealen, intermittierenden Druckbeatmung, bestehend aus einer steuerbaren Druckgasquelle mit Atemgas, einem mit dieser verbundenem Handstück, ein im Handstück angeordnetes kurzes Hauptrohr mit verschließbarer Ausströmöffnung in die Atmosphäre, einer im Hauptrohr zentrisch angeordneten Venturidüse zur Unterstützung der Expiration und eine seitlich am Hauptrohr, in Strömungsrichtung vor der Venturidüse, abzweigendes Beatmungsrohr für die Zu- und Abfuhr des Atemgases zum/vom Patienten und einem Druckmessgerät für den Gasdruck, wobei an der Ausströmöffnung ein manuell betätigbarer Geber zur Betätigung eines Drosselventils für die Minderung des Drucks und/oder der Durchflussmenge des aus der Druckgasquelle zur Venturidüse strömenden Atemgases bzw. Druckgases während der Inspirationsphase angeordnet ist, wobei unmittelbar am Beatmungsrohr, im Bereich zwischen dem Katheter und der Venturidüse ein Druckmessgerät angeschlossen ist und wobei zwischen dem Druckmessgerät und der Venturidüse ein Schließventil angeordnet ist, dass für eine momentane, exakte Druckmessung des Lungendrucks manuell betätigbar ist, und wobei zwischen dem Schließventil und dem Druckmessgerät ein betätigbares oder automatisches Sicherheitsventil gegen eine Drucküberschreitung des Beatmungsdrucks angeschlossen ist.

Die Gasstromumkehrelemente erlauben insbesondere eine vollständige Beatmung eines Patienten (d.h. es kann ausreichend Atemluft zugeführt und verbrauchte Atemluft wieder abgeführt werden - Normoventilation) über eine unbegrenzte Zeitdauer. Dabei durchströmt der Gasstrom in beiden Richtungen (hin zu und weg von den Atemwegen des Patienten) ein einziges Lumen.

Bei der Beatmung (Normoventilation), insbesondere mittels der Gasstromumkehrelemente, ist es erforderlich, den Druck innerhalb der Atemwege zu bestimmen und zu überwachen. So kann eine ausreichende Beatmung sichergestellt und Verletzungen der Atemwege (z. B. Barotrauma) können vermieden werden.

Über das Gasstromumkehrelement und über den dünnen Katheter/das dünne Lumen wird ein nicht unerheblicher Druckverlust hervorgerufen, der abhängig ist von zahlreichen Größen (anliegender Druck, Volumenstrom, Durchmesser Lumen, Länge Lumen, usw.). Dieser Druckverlust muss bei einer Messung des Drucks der Atemwege ggf. berücksichtigt werden (wenn die Druckmessung noch vor dem Gasstromumkehrelement - also nicht zwischen Gasstromumkehrelement und Atemwegen - erfolgt).

Für die Druckmessung werden Druckmessgeräte, z. B. Manometer, auch direkt mit dem Lumen, dass das Gasstromumkehrelement mit den Atemwegen des Patienten verbindet, oder mit dem Gasstromumkehrelement selbst verbunden (bei den bekannten Gasstromumkehrelementen mit z. B. mit dem Abzweigungsstück, also mit dem Bereich des Gasstromumkehrelementes, durch den Atemluft zugeführt und verbrauchte Atemluft abgeführt wird). Dabei werden die Druckmessgeräte kontinuierlich mit den wechselnden Druckverhältnissen in der angeschlossenen Leitung (Lumen/Abzweigungsstück) beaufschlagt. Diese Druckverhältnisse variieren gerade bei dem Einsatz von Gasstromumkehrelementen zwischen jedem Ein- und Ausatmungsvorgang von 0,5 bar und bis zu 5 bar.

Diese Druckschwankungen können dazu führen, dass die eingesetzten Druckmessgeräte dekalibriert oder sogar zerstört werden. In jedem Fall ist aber ein Bestimmen des in den Atemwegen vorliegenden Drucks nur schwer möglich bzw. unmöglich.

Die US 5,669,380 A offenbart eine Beatmungsvorrichtung, wobei mit einer Druckmesseinrichtung der Ventilationsvorgang überwacht werden kann.

Die EP 0 130 930 A ist auf ein Druckmessverfahren bei einer endotrachealen Beatmung gerichtet. Dafür wird eine Ventilanordnung beschrieben.

Hiervon ausgehend liegt der vorliegenden Erfindung die Zielsetzung zugrunde, die aus dem Stand der Technik bekannten Nachteile zumindest teilweise zu überwinden und eine Anordnung zu schaffen, mit der eine einfache und sichere Druckmessung erfolgen kann. Eine Beschädigung oder Dekalibrierung der eingesetzten Messgeräte soll verhindert werden.

Diese Zielsetzung wird durch eine Anordnung mit den Merkmalen des Patentanspruchs 1 erreicht. Vorteilhafte Weiterbildungen und Ausgestaltungen der Anordnung sind Gegenstand der jeweils abhängigen Ansprüche.

Die Erfindung betrifft eine Anordnung, umfassend einen schaltbaren Anschluss und ein Gasstromumkehrelement, das zur Ausnutzung eines unter Überdruck stehenden Gasvorrates, insbesondere Sauerstoff oder ein anderes zur Beatmung und/ oder zur geplanten Behandlung geeignetes Gas, zur wahlweisen Erzeugung eines Gasstromes von oder zu einem Leitungsanschluss, der über ein Lumen der Anordnung mit einem Atemweg eines Patienten verbindbar ist, geeignet ist. Das Gasstromumkehrelement ist zumindest ausgebildet als ein Hauptstück mit Abzweigungsstück, wobei das Hauptstück einen Druckanschluss zum Anschluss an den Gasvorrat mit mindestens einer verschließbaren Auslassöffnung verbindet und das Abzweigungsstück mit einer ersten Leitung das Hauptstück mit dem Leitungsanschluss verbindet. In dem Hauptstück ist eine Düse so ausgebildet und angeordnet, dass durch einen von dem Druckanschluss durch die Düse zu der Auslassöffnung strömenden Gasstrom im Hauptstück bei geöffneter Auslassöffnung auch ein Gasstrom durch die erste Leitung in Richtung Auslassöffnung erzeugbar ist. Insbesondere sind auch Gasstromumkehrelemente gemäß WO 2008/113752 A1 oder US 61/844746 hier einsetzbar. Der schaltbare Anschluss ist an dem Abzweigungsstück oder an dem Leitungsanschluss angeordnet und verbindet zumindest eine zweite Leitung der Anordnung schaltbar mit der ersten Leitung, wobei der schaltbare Anschluss zumindest zwei Schaltpositionen aufweist, wobei
(1) in der ersten Schaltposition die erste Leitung geöffnet und die zweite Leitung verschlossen ist, so dass der Gasstrom durch den schaltbaren Anschluss in beiden Richtungen entlang der ersten Leitung strömen kann;
(2) in der zweiten Schaltposition, wenn der schaltbare Anschluss an dem Abzweigungsstück angeordnet ist, die zweite Leitung über den schaltbaren Anschluss strömungstechnisch mit dem Leitungsanschluss und mit dem Lumen, über das der Leitungsanschluss mit dem Atemweg des Patienten verbindbar ist, verbunden ist oder, wenn der schaltbare Anschluss an dem Leitungsanschluss angeordnet ist, die zweite Leitung strömungstechnisch mit dem Lumen, über das der Leitungsanschluss mit dem Atemweg des Patienten verbindbar ist, verbunden ist.

Erfindungsgemäß ist in der zweiten Schaltposition die erste Leitung hin zu dem Hauptstück verschlossen und die zweite Leitung über den schaltbaren Anschluss strömungstechnisch nur mit dem Leitungsanschluss und/oder mit dem Lumen, über das der Leitungsanschluss mit dem Atemweg des Patienten verbindbar ist, verbunden, so dass zu keinem Zeitpunkt die zweite Leitung strömungstechnisch mit dem Hauptstück verbindbar ist.

Insbesondere ist das Gasstromumkehrelement in einem ergonomisch geformten Gehäuse angeordnet, das in eine Hand eines Menschen angepasst ist, wobei der schaltbare Anschluss manuell, vorzugsweise mit mindestens einem Finger derselben Hand eines Menschen, zumindest von der ersten Schaltposition in die zweite Schaltposition überführbar ist.

Bevorzugt ist der Anschluss über ein Hebelelement betätigbar.

Insbesondere ist das Hebelelement zur Betätigung des Anschlusses drehbar an dem Anschluss angeordnet.

Gemäß einer bevorzugten Ausgestaltung wird das Hebelelement für einen Schaltvorgang zwischen der ersten Schaltposition und der zweiten Schaltposition um im Wesentlichen eine viertel Umdrehung (also um ca. 90 Winkelgrad) gedreht.

Insbesondere erfolgt zumindest ein Schaltvorgang von der zweiten Schaltposition in die erste Schaltposition selbsttätig. Selbsttätig heißt hier insbesondere, dass eine Betätigung z. B. des Hebelelements für diesen Schaltvorgang nicht notwendig ist. Es sind insbesondere Rückstellelemente in dem Anschluss integriert (z. B. Federn oder ähnliches), die diesen Schaltvorgang vornehmen. Das bedeutet insbesondere, dass der Anschluss bei Nichtbetätigung immer in der ersten Schaltposition angeordnet ist.

Insbesondere ist der Anschluss eine Art Weiche, die bestimmte Leitungen (hier erste Leitung hin zum Hauptstück; zweite Leitung, z. B. hin zur Druckmesseinrichtung; und erste Leitung bzw. Lumen hin zum Patienten) miteinander strömungstechnisch verbindet oder die Verbindung zwischen den Leitungen verschließt. Insbesondere sind innerhalb des Anschlusses dafür Durchleitungen vorgesehen, wobei die Durchleitungen z. B. durch Drehung in unterschiedlicher Weise (erste und zweite Schaltposition) mit den, an den Anschluss angebundenen, Leitungen verbunden werden können.

Bevorzugt sind der Anschluss und das Gasstromumkehrelement lösbar miteinander verbindbar. D. h. insbesondere, dass Anschluss und Gasstromumkehrelement jeweils getrennt voneinander vorliegen und z. B. über eine sogenannte "Luer-Lock"-Verbindung miteinander verbunden werden.

Andererseits ist es auch vorteilhaft, dass der Anschluss und das Gasstromumkehrelement unlösbar (einteilig; stoffschlüssig) miteinander verbunden sind. D. h. insbesondere, dass Anschluss und Gasstromumkehrelement nur zerstörend voneinander getrennt werden können. Eine solche einteilige Ausführung kann insbesondere durch einen Nutzer einfacher gehandhabt und ggf. kostengünstiger hergestellt werden.

Insbesondere ist zumindest eine Verbindung aus der Gruppe
- Anschluss mit Leitungsanschluss,
- Anschluss mit zweiter Leitung sowie
- Anschluss mit erster Leitung
als eine sogenannte "Luer Lock" Verbindung ausgeführt.

Es wird weiter ein nicht erfindungsgemäßes Verfahren zum Messen eines Drucks in einer erfindungsgemäßen Anordnung vorgeschlagen, wobei an der zweiten Leitung eine Druckmesseinrichtung (z. B. ein Manometer) angeordnet/angeschlossen ist, wobei, wenn der schaltbare Anschluss sich in der zweiten Schaltposition befindet, der Druck in der ersten Leitung zwischen Anschluss und Leitungsanschluss oder in dem Lumen zwischen Anschluss und insbesondere einem Patienten über die Druckmesseinrichtung gemessen werden kann.

Zur Sicherheit des Patienten ist es wichtig, dass nicht schon bei einer versehentlichen Manipulation am Gasstromumkehrelement irgendein Einfluss auf den Atemweg ausgeübt wird. Daher ist es vorteilhaft, wenn vorzugsweise in dem Abzweigungsstück mindestens eine Sicherheitsöffhung vorhanden ist (siehe WO 2008/113752 A1). Bevorzugt sollten sogar zwei, besonders bevorzugt zwei nebeneinander oder einander gegenüberliegende Sicherheitsöffhungen vorhanden sein. Dabei dienen diese Sicherheitsöffhungen gleichzeitig der Steuerung des Über- oder Unterdruckes in Richtung Patient. Bei Freigabe wirken sie zudem als Druckausgleichsöffnungen und ermöglichen den Ausgleich eines intrathorakalen Über- oder Unterdruckes, wenn man die Jet-Beatmung kurz pausiert. Dadurch kann sich der Druck in der Lunge mit dem Außendruck äquilibrieren. Dies ist insbesondere dann wichtig, wenn man einen komplett verlegten Atemweg hat, und man nicht weiß, ob die Lunge des Patienten aktuell etwas zu stark aufgeblasen oder leer gesaugt ist (insbesondere wenn Druckmessung zu diesem Zeitpunkt nicht erfolgt oder kein Druckmesser angeschlossen ist).

Bei Verbinden des Druckanschlusses des Gasstromumkehrelementes mit einer Druckgasquelle strömt ein Gasstrom durch die Düse zur Auslassöffnung, was ohne Sicherheitsöffnung(en) direkt zu einem Unterdruck am Leitungsanschluss und damit eventuell auch im Atemweg führt. Durch die noch offenen Sicherheitsöffnung(en), die dazu einen genügend großen Querschnitt haben sollten, wird dies vermieden, da der Unterdruck durch dort einströmende Umgebungsluft ausgeglichen wird. Erst wenn alle Sicherheitsöffhungen gezielt, z.B. durch die Finger eines Menschen/ einer Bedienperson, geschlossen werden, entsteht am Leitungsanschluss ein Unterdruck.

Durch zusätzliches Schließen der Auslassöffnung kann dann ein Gas- oder Gasgemischstrom auch in den Atemweg geleitet werden. Bei Freigabe der Sicherheitsöffnungen entweicht der Gas- oder Gasgemischstrom hingegen sofort quantitativ in die Umgebungsluft. Durch wechselweises Öffnen und Schließen der Auslassöffnung bei geschlossenen Sicherheitsöffnungen erreicht man eine wirkungsvolle Beatmung.

Der Anschluss bewirkt insbesondere, dass der Druck in den Atemwegen eines Patienten (überhaupt) bestimmt werden kann, wobei das Druckmessgerät nicht mit starken Druckschwankungen beaufschlagt wird.

Insbesondere ist die Anordnung so gestaltet, dass ein Schließen von Auslassöffnung und Sicherheitsöffnungen und ein gleichzeitiges Vorliegen der zweiten Schaltposition verhindert wird oder nur durch bewusste Bedienung erreichbar ist.

Zu keinem Zeitpunkt ist die zweite Leitung strömungstechnisch mit dem Hauptstück verbindbar. Das wird dadurch erreicht, dass in der zweiten Schaltposition die erste Leitung hin zu dem Hauptstück verschlossen ist und die zweite Leitung über den Anschluss strömungstechnisch nur mit dem Leitungsanschluss und/oder mit dem Lumen verbunden ist.

Damit wird insbesondere sichergestellt, dass die Druckmesseinrichtung nicht mit der Druckgasquelle strömungstechnisch verbunden wird. Eine Beaufschlagung mit hohen Drücken (z. B. mehr als 1,5 bar) wird somit nicht auftreten oder nur dann, wenn eine bewusste Bedienung der Anordnung erfolgt.

Besonders bei einer Notfallversorgung ist es vorteilhaft, die Auslassöffnung und die Sicherheitsöffhungen so anzuordnen, dass sie manuell, vorzugsweise mit Handballen und/oder Fingern nur einer Hand eines Menschen oder einer Bedienperson wahlweise einzeln oder gemeinsam ganz oder teilweise verschließbar sind. So kann ein Mensch mit einer Hand die Beatmung nach Bedarf durchführen, unterbrechen oder durch teilweises Verschließen der Öffnungen regulieren, hat aber noch die andere Hand für weitere Maßnahmen oder Tätigkeiten (z.B. Sicherung des Katheters oder der Kanüle) frei.

Insbesondere ist/sind die Sicherheitsöffnung/en so angeordnet, dass sie besonders einfach mit einem bestimmten Finger bedient/verschlossen werden können (z. B. mit dem Zeigefinger, während die Auslassöffnung z. B. mit dem Daumen bedient/verschlossen wird). Insbesondere muss für eine Betätigung des Anschlusses, also zur Vornahme eines Schaltvorgangs, derselbe Finger verwendet werden, der auch für die Bedienung der Sicherheitsöffnung eingesetzt wird. Der Finger muss also, zur Bedienung des Anschlusses, von der Sicherheitsöffnung entfernt werden. Die Sicherheitsöffnung ist dann offen und der Gasstrom entweicht in die Umgebung. Damit wird verhindert, dass die Druckmesseinrichtung mit dem Druck des Gasstroms aus der Druckgasquelle strömungstechnisch verbunden wird.

Für den Einsatz der erfindungsgemäßen Anordnung werden lediglich eine Atemluftdruckflasche oder ein stationärer Druckgasanschluss (z. B. in einem Krankenhaus) und eine Verbindungsleitung benötigt.

Die Ausführungen zu dem Verfahren sind auf die Anordnung übertragbar und umgekehrt. Bevorzugte Ausführungsbeispiele der Erfindung, auf die diese jedoch nicht beschränkt ist, werden im Folgenden anhand der Zeichnungen näher erläutert. Gleiche Bezugszeichen zeigen gleiche Gegenstände. Es zeigen:
- Fig. 1:: einen Längsschnitt durch eine Anordnung mit schematisch dargestellten Peripheriegeräten;
- Fig. 2:: einen schaltbaren Anschluss in der zweiten Schaltposition;
- Fig. 3:: schematisch eine Anordnung im Schnitt;
- Fig. 4:: schematisch ein Gasstromumkehrelement mit Bypass im Schnitt;
- Fig. 5:: schematisch das Gasstromumkehrelement aus Fig. 4 im Schnitt mit einer anderen Stellung des Schließelements;
- Fig. 6:: schematisch das Gasstromumkehrelement aus Fig. 4 und 5 im Schnitt mit einer weiteren Stellung des Schließelements und verschlossener Auslassöffnung.

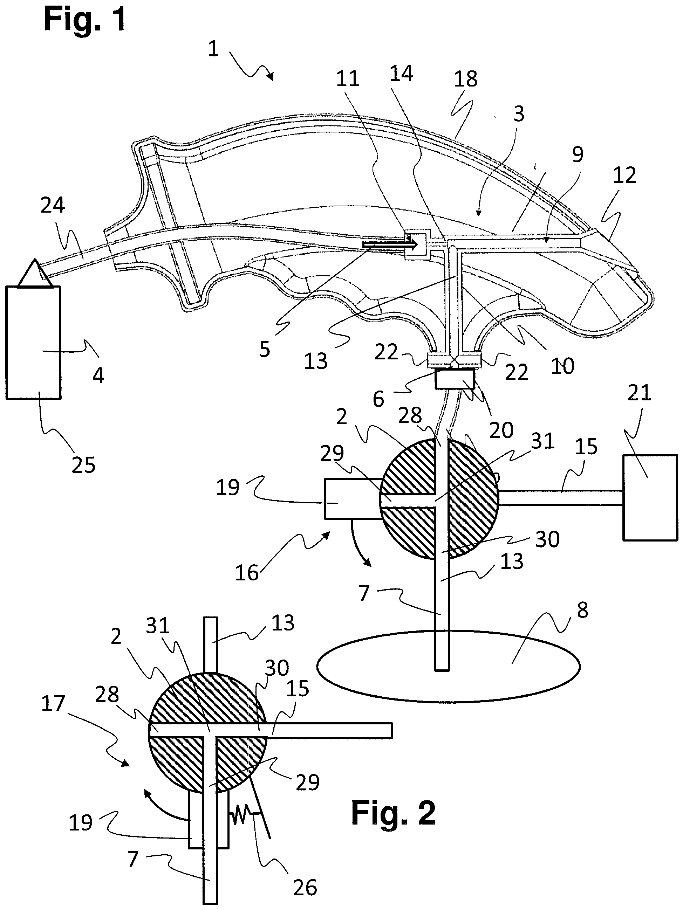

Fig. 1 zeigt ein Gasstromumkehrelement 3 mit einem Hauptstück 9, welches einen Druckanschluss 11 mit einer Auslassöffnung 12 verbindet. Der Druckanschluss 11 kann über eine Verbindungsleitung 24 mit einem unter Überdruck stehenden Gasvorrat 4 in einer Druckgasquelle 25 verbunden werden. Im Allgemeinen steht für die Notfallversorgung von Patienten eine Sauerstoffdruckflasche zur Verfügung. Die Druckgasquelle kann auch über einen stationären Anschluss (z. B. in einem Krankenhaus) bereitgestellt werden. Von dem Hauptstück 9 zweigt ein Abzweigungsstück 10 ab, welches zu einem Leitungsanschluss 6 führt. In dem Hauptstück 9 ist eine Düse 14 ausgebildet, durch die ein Gasstrom 5 vom Druckanschluss 11 zur Auslassöffnung 12 strömen kann, wobei diese Düse 14 in der Nähe des Abzweigungsstückes 10 liegt, so dass der durch die Düse 14 zur Auslassöffnung 12 strömende Gasstrom 5 einen Unterdruck im Abzweigungsstück 10 erzeugt.

Weiter weist das Abzweigungsstück 10 zwei Sicherheitsöffhungen 22 auf. Solange diese beiden Sicherheitsöffnungen 22 nicht gezielt verschlossen werden, übt die Anordnung 1 auch bei strömendem Gasstrom 5 keinen Einfluss auf den Atemweg eines Patienten 8 aus. Nur wenn diese Sicherheitsöffnungen 22 verschlossen werden, entsteht im Abzweigungsstück 10 ein Unterdruck, solange die Auslassöffnung 12 offen ist, und ein Überdruck, wenn die Auslassöffnung 12 verschlossen wird. Bevorzugt sollten alle Öffnungen 12, 22 so angeordnet sein, dass sie von einer Bedienperson zwar mit einer Hand bewusst, aber kaum versehentlich verschlossen werden können. Zumindest die Auslassöffnung 12 sollte dann mit einem Finger (z. B. durch den Daumen) verschließbar sein, da sie bei der Beatmung wechselweise geöffnet und geschlossen werden muss.

An den Leitungsanschluss 6 ist hier der schaltbare Anschluss angebunden, bevorzugt über eine sogenannte "Luer Lock"-Verbindung 20. Über diese Verbindung 20 sind der Anschluss 2 und das Gasstromumkehrelement 3 lösbar miteinander verbunden.

Die ganze Anordnung 1 ist vorzugsweise aus Kunststoff hergestellt.

Der schaltbare Anschluss 2 ist hier unmittelbar an dem Leitungsanschluss 6 (zwischen Leitungsanschluss 6 und dem Patienten 8) am Gehäuse 18 angeordnet und verbindet eine zweite Leitung 15 schaltbar mit der ersten Leitung 13, wobei der schaltbare Anschluss 2 hier in der ersten Schaltposition 16 dargestellt ist. Dabei ist die erste Leitung 13 geöffnet und die zweite Leitung 15 verschlossen, so dass der Gasstrom 5 durch den Anschluss 2 in beiden Richtungen entlang der erste Leitung 13 strömen kann. Eine Druckmesseinrichtung 21 ist mit der zweiten Leitung 15 verbunden. Ein Lumen 7 verbindet Anschluss 2 und die Atemwege des Patienten 8. Der Anschluss 2 weist hier eine T-förmige Verbindungsleitung 31 auf. Die T-förmige Verbindungsleitung 31 erstreckt sich zwischen einem ersten Ende 28, einem zweiten Ende 29 und einem dritten Ende 31. In der gezeigten ersten Schaltposition 16 ist das erste Ende 28 an dem Leitungsanschluss 6 und das dritte Ende 30 an dem Lumen 7 angeordnet, so dass ein Gasstrom 5 entlang der ersten Leitung 13 durch den Leitungsanschluss 6, die Verbindungsleitung 31 und dem Lumen 7 strömen kann. Das zweite Ende 29 ist hier verschlossen (z. B. über ein Gehäuse des Anschlusses 2).

Der Anschluss 2 ist über ein Hebelelement 19 betätigbar. Das Hebelelement 19 ist zur Betätigung des Anschlusses 2 drehbar an dem Anschluss 2 angeordnet. Das Hebelelement 19 wird für einen Schaltvorgang zwischen der ersten Schaltposition 16 und der zweiten Schaltposition 17 (siehe Fig. 2) um im Wesentlichen eine viertel Umdrehung (90 Winkelgrad) gedreht (siehe Drehrichtung in Fig. 1 und 2).

Das Gasstromumkehrelement 3 ist in einem ergonomisch geformten Gehäuse 18 angeordnet. Das Gehäuse 18 ist dabei so geformt, dass es an die menschliche Hand angepasst ist. Die Sicherheitsöffhungen 22 lassen sich mit den Fingern, die Auslassöffnung 12 lässt sich mit dem Daumen ganz oder teilweise verschließen. Der Anschluss 2 ist im oder wie hier gezeigt in unmittelbarer Nähe des Gehäuses 18 angeordnet und lässt sich insbesondere über Finger derselben Hand über das Hebelelement 19 betätigen. Durch eine derartige Ausgestaltung ist die Bedienung der Anordnung 1 mit nur einer Hand eines Menschen möglich. Zur einfacheren Handhabung kann auch nur eine Sicherheitsöffnung 22 vorgesehen werden. Das Gehäuse 18 und der Anschluss 2 können in verschiedener Weise zusammengesetzt oder zusammenhängend hergestellt werden. Wichtig ist, dass insbesondere eine leicht manuell zu bedienende Anordnung 1 entsteht, die in einer Hand gehalten werden kann, wobei die Auslassöffnung 12 und die Sicherheitsöffnungen 22 mit den Fingern oder anderen Teilen dieser Hand verschlossen werden können und wobei der Anschluss 2 über Finger derselben Hand (insbesondere durch denselben Finger, der die Sicherheitsöffnung 22 betätigt) betätigbar ist.

Fig. 2 zeigt einen schaltbaren Anschluss 2 in der zweiten Schaltposition 17. Der Anschluss 2 weist eine Rückstelleinrichtung 26 (z. B. eine Druckfeder) auf, so dass der Schaltvorgang von der zweiten Schaltposition 17 zurück in die erste Schaltposition 16 selbsttätig erfolgt. In der hier gezeigten zweiten Schaltposition 17 ist die zweite Leitung 15 über das dritte Ende 30, die Verbindungsleitung 31 und das zweite Ende 29 des Anschlusses 2 strömungstechnisch nur mit dem Lumen 7 verbunden, so dass die Druckmesseinrichtung 21 den Druck in dem Lumen 7 messen kann und damit der Druck in den Atemwegen eines Patienten 8 bestimmbar ist. Der Anschluss 2 verbindet in der hier gezeigten zweiten Schaltposition 17 ausschließlich die zweite Leitung 15 mit dem Lumen 7. Das erste Ende 28 ist hier verschlossen (z. B. über ein Gehäuse des Anschlusses 2).

Hier ist gezeigt, dass durch den Anschluss 2 in der zweiten Schaltposition 17 die erste Leitung 13 hin zu dem Hauptstück 9 verschlossen ist und die zweite Leitung 15 über den Anschluss 2 strömungstechnisch nur mit dem Lumen 7 verbunden ist.

Alternativ ist es möglich den Anschluss 2 in das Gehäuse 18 des Gastromumkehrelements 3 zu integrieren. Dann ist z. B. der schaltbare Anschluss 2 an dem Abzweigungsstück 10 angeordnet, also zwischen Hauptstück 3 und Leitungsanschluss 6 (für ein Lumen 7, z. B. ein Katheter) an die erste Leitung 13 angebunden. Die erste Leitung 13 wird durch den Anschluss 2 geöffnet oder geschlossen, wobei die zweite Leitung 15 über die erste Leitung 13 und den Leitungsanschluss 6 mit dem Lumen 7 verbindbar ist.

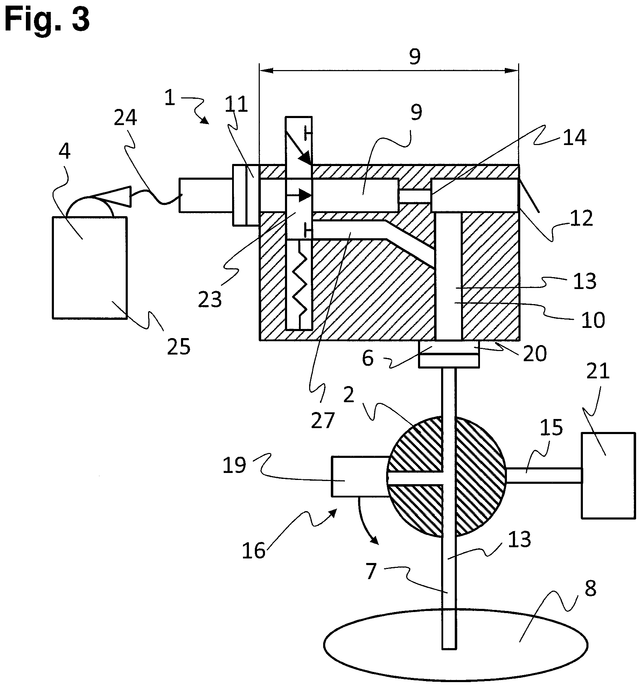

Fig. 3 zeigt schematisch eine Anordnung 1 im Schnitt. Diese Anordnung 1 umfasst ein Gaststromumkehrelement 3 z. B. gemäß US 61/844746. Hier wird ebenfalls ein unter Überdruck stehender Gasvorrat 4 zur wahlweisen Erzeugung eines Gasstromes 5 von oder zu dem Leitungsanschluss 6, verwendet. Das Gasstromumkehrelement 3 umfasst ein Hauptstück 9 mit Abzweigungsstück 10, wobei das Hauptstück 9 einen Druckanschluss 11 zum Anschluss an den Gasvorrat 4 mit mindestens einer verschließbaren Auslassöffnung 12 verbindet und das Abzweigungsstück 10 mit einer ersten Leitung 13 das Hauptstück 9 mit dem Leitungsanschluss 6 verbindet. In dem Hauptstück 9 ist eine Düse 14 so ausgebildet und angeordnet, dass durch einen von dem Druckanschluss 11 durch die Düse 14 zu der Auslassöffnung 12 strömenden Gasstrom 5 im Hauptstück 9 bei geöffneter Auslassöffnung 12 auch ein Gasstrom 5 durch die erste Leitung 13 in Richtung Auslassöffnung 12 erzeugbar ist. Hier ist ein Schließelement 23 (z. B. ein schaltbares Ventil oder eine Klemmung, die von außen eine flexible Leitung zusammendrücken und verschließen kann) in dem Hauptstück 9 angeordnet, so dass ggf. die Düse 14 umgangen und über den Bypass 27 der Gasstrom 5 direkt dem Leitungsanschluss 6 zugeführt werden kann. Auch hier ist der schaltbare Anschluss 2 an dem Leitungsanschluss 6 angeordnet und zumindest eine zweite Leitung 15 mit der ersten Leitung 13 schaltbar verbindbar.

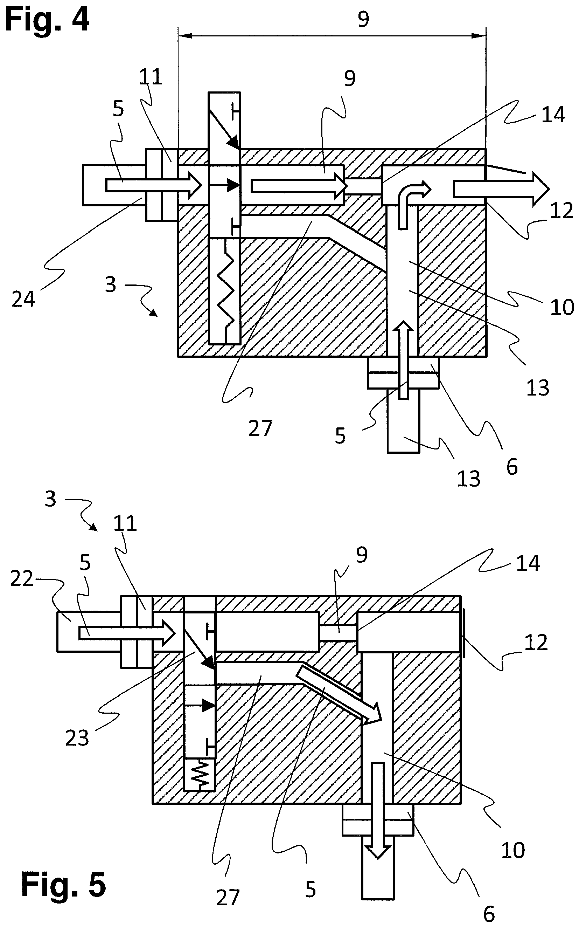

Fig. 4 zeigt schematisch ein Gasstromumkehrelement 3 mit Bypass 27 (z. B. gemäß US 61/844746, und gemäß Fig. 3) im Schnitt. Infolge der Betätigung des Schließelements 23 strömt hier Gasstrom 5 vom Druckanschluss 11 in Richtung Auslassöffnung 12 und erzeugt damit einen weiteren Gasstrom 5, der über den Leitungsanschluss 6 durch das Abzweigungsstück 10 hin zur Auslassöffnung 12 strömt.

Fig. 5 zeigt schematisch das Gasstromumkehrelement 3 aus Fig. 4 im Schnitt mit einer anderen Stellung des Schließelements 23. Hier wird der Gasstrom 5, der über den Druckanschluss 11 in das Gaststromumkehrelement 3 einströmt über den Bypass 27 direkt in die erste Leitung 13 im Abzweigungsstück 10 überführt, ohne dass ein Druckverlust durch Überströmung der Düse 14 auftritt.

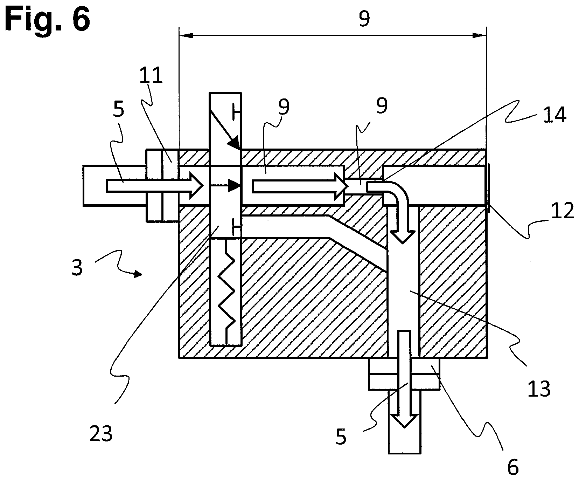

Fig. 6 zeigt schematisch das Gasstromumkehrelement 3 aus Fig. 4 und 5 im Schnitt mit einer weiteren Stellung des Schließelements 23 und verschlossener Auslassöffnung 12. Hier wird der Gasstrom 5, der über den Druckanschluss 11 in das Gaststromumkehrelement 3 einströmt über das Hauptstück 9 und die Düse 14 in die erste Leitung 13 im Abzweigungsstück 10 überführt.

### Bezugszeichenliste

- 1: Anordnung
- 2: Anschluss
- 3: Gasstromumkehrelement
- 4: Gasvorrat
- 5: Gasstrom
- 6: Leitungsanschluss
- 7: Lumen
- 8: Patient
- 9: Hauptstück
- 10: Abzweigungsstück
- 11: Druckanschluss
- 12: Auslassöffnung
- 13: erste Leitung
- 14: Düse
- 15: zweite Leitung
- 16: erste Schaltposition
- 17: zweite Schaltposition
- 18: Gehäuse
- 19: Hebelelement
- 20: Verbindung
- 21: Druckmesseinrichtung
- 22: Sicherheitsöffnung
- 23: Schließelement
- 24: Verbindungsleitung
- 25: Druckgasquelle
- 26: Rückstelleinrichtung
- 27: Bypass
- 28: Erstes Ende
- 29: Zweites Ende
- 30: drittes Ende
- 31: Verbindungsleitung

## Patentansprüche

1. Anordnung (1), umfassend einen schaltbaren Anschluss (2) und ein Gasstromumkehrelement (3), das zur Ausnutzung eines unter Überdruck stehenden Gasvorrates (4), zur wahlweisen Erzeugung eines Gasstromes (5) von oder zu einem Leitungsanschluss (6), der über ein Lumen (7) der Anordnung (1) mit einem Atemweg eines Patienten (8) verbindbar ist, geeignet ist; wobei das Gasstromumkehrelement (3) zumindest ausgebildet ist als ein Hauptstück (9) mit Abzweigungsstück (10), wobei das Hauptstück (9) einen Druckanschluss (11) zum Anschluss an den Gasvorrat (4) mit mindestens einer verschließbaren Auslassöffnung (12) verbindet und das Abzweigungsstück (10) mit einer ersten Leitung (13) das Hauptstück (9) mit dem Leitungsanschluss (6) verbindet, wobei in dem Hauptstück (9) eine Düse (14) so ausgebildet und angeordnet ist, dass durch einen von dem Druckanschluss (11) durch die Düse (14) zu der Auslassöffnung (12) strömenden Gasstrom (5) im Hauptstück (9) bei geöffneter Auslassöffnung (12) auch ein Gasstrom (5) durch die erste Leitung (13) in Richtung Auslassöffnung (12) erzeugbar ist, wobei der schaltbare Anschluss (2) an dem Abzweigungsstück (10) oder an dem Leitungsanschluss (6) angeordnet ist und zumindest eine zweite Leitung (15) der Anordnung (1) mit der ersten Leitung (13) schaltbar verbindet, wobei der schaltbare Anschluss (2) zumindest zwei Schaltpositionen (16, 17) aufweist, wobei:
1) in der ersten Schaltposition (16) die erste Leitung (13) geöffnet und die zweite Leitung (15) verschlossen ist, so dass der Gasstrom (5) durch den schaltbaren Anschluss (2) in beiden Richtungen entlang der ersten Leitung (13) strömen kann;
2) in der zweiten Schaltposition (17), wenn der schaltbare Anschluss (2) an dem Abzweigungsstück (10) angeordnet ist, die zweite Leitung (15) über den schaltbaren Anschluss (2) strömungstechnisch mit dem Leitungsanschluss (6) und mit dem Lumen (7), über das der Leitungsanschluss (6) mit dem Atemweg des Patienten verbindbar ist, verbunden ist oder, wenn der schaltbare Anschluss (2) an dem Leitungsanschluss (6) angeordnet ist, die zweite Leitung (15) strömungstechnisch mit dem Lumen (7), über das der Leitungsanschluss (6) mit dem Atemweg des Patienten verbindbar ist, verbunden ist; und
in der zweiten Schaltposition (17) die erste Leitung (13) hin zu dem Hauptstück (9) verschlossen und die zweite Leitung (15) über den schaltbaren Anschluss (2) strömungstechnisch nur mit dem Leitungsanschluss und/oder mit dem Lumen (7), über das der Leitungsanschluss (6) mit dem Atemweg des Patienten verbindbar ist, verbunden ist, so dass zu keinem Zeitpunkt die zweite Leitung (15) strömungstechnisch mit dem Hauptstück (9) verbindbar ist.

2. Anordnung (1) nach Anspruch 1, wobei das Gasstromumkehrelement (3) in einem ergonomisch geformten Gehäuse (18) angeordnet ist, das in eine Hand eines Menschen angepasst ist, wobei der schaltbare Anschluss (2) manuell, vorzugsweise mit mindestens einem Finger derselben Hand eines Menschen, zumindest von der ersten Schaltposition (16) in die zweite Schaltposition (17) überführbar ist.

3. Anordnung (1) nach einem der vorhergehenden Ansprüche, wobei der Anschluss (2) über ein Hebelelement (19) betätigbar ist.

4. Anordnung (1) nach Anspruch 3, wobei das Hebelelement (19) zur Betätigung des Anschlusses (2) drehbar an dem Anschluss (2) angeordnet ist.

5. Anordnung (1) nach Anspruch 4, wobei für einen Schaltvorgang zwischen der ersten Schaltposition (16) und der zweiten Schaltposition (17) eine Drehung des Hebelelements (19) um eine viertel Umdrehung erforderlich ist.

6. Anordnung (1) nach einem der vorhergehenden Ansprüche, wobei zumindest ein Schaltvorgang von der zweiten Schaltposition (17) in die erste Schaltposition (16) selbsttätig durchführbar ist.

7. Anordnung (1) nach einem der vorhergehenden Ansprüche, wobei der schaltbare Anschluss (2) und das Gasstromumkehrelement (3) lösbar miteinander verbindbar sind.

8. Anordnung (1) nach einem der Ansprüche 1 bis 6, wobei der schaltbare Anschluss (2) und das Gasstromumkehrelement (3) unlösbar miteinander verbunden sind.

9. Anordnung (1) nach einem der vorhergehenden Ansprüche, wobei zumindest eine Verbindung (20) aus der Gruppe schaltbarer Anschluss (2) mit Leitungsanschluss (6), schaltbarer Anschluss (2) mit zweiter Leitung (15) sowie schaltbarer Anschluss (2) mit erster Leitung (13) als eine sogenannte "Luer Lock" Verbindung ausgeführt ist.

## Claims

1. Arrangement (1) comprising a switchable connector (2) and a gas flow reversing element (3) which is suitable for exploiting a gas supply (4) under positive pressure for selectively generating a gas flow (5) from or to a line connector (6) which is connectable to airways of a patient (8) by way of a lumen (7) of the arrangement (1); wherein the gas flow reversing element (3) is embodied at least as a main piece (9) with a branching piece (10), wherein the main piece (9) connects a pressure connector (11) for connection to the gas supply (4) with at least one closable outlet opening (12) and the branching piece (10) connects, by way of a first line (13), the main piece (9) to the line connector (6), wherein a nozzle (14) is embodied and arranged in the main piece (9) in such a way that a gas flow (5) flowing from the pressure connector (11) through the nozzle (14) to the outlet opening (12) also is able to produce a gas flow (5) through the first line (13) in the direction of the outlet opening (12) in the main piece (9) if the outlet opening (12) is open, wherein the switchable connector (2) is arranged at the branching piece (10) or at the line connector (6) and switchably connects at least a second line (15) of the arrangement (1) to the first line (13), wherein the switchable connector (2) has at least two switching positions (16, 17), wherein,
(1) in the first switching position (16), the first line (13) is open and the second line (15) is closed such that the gas flow (5) can flow through the switchable connector (2) in both directions along the first line (13);
(2) in the second switching position (17), if the switchable connector (2) is arranged at the branching piece (10), the second line (15) is flow-connected via the switchable connector (2) to the line connector (6) and to the lumen (7), by which the line connector (6) is connectable to the airways of a patient, or, if the switchable connector (2) is arranged at the line connector (6), the second line (15) is flow-connected to the lumen (7), by which the line connector (6) is connectable to the airways of a patient; and
in the second switching position (17) the first line (13) in the direction to the main piece (9) is closed and the second line (15) is flow-connected via the switchable connector (2) only to the line connector (6) and/or to the lumen (7), by which the line connector (6) is connectable to the airways of a patient, so that at no point in time the second line (15) is flow-connectable to the main piece (9).

2. Arrangement (1) as claimed in claim 1, wherein the gas flow reversing element (3) is arranged in an ergonomically shaped housing (18), which is adapted to a hand of a human, wherein the switchable connector (2) can be transferred at least from the first switching position (16) to the second switching position (17) manually, preferably using at least one finger of the same hand of a human.

3. Arrangement (1) as claimed in any one of the preceding claims, wherein the connector (2) is actuatable by way of a lever element (19).

4. Arrangement (1) as claimed in claim 3, wherein the lever element (19) is rotatably arranged at the connector (2) for the purposes of actuating the switchable connector (2).

5. Arrangement (1) as claimed in claim 4, wherein, for a switching process between the first switching position (16) and the second switching position (17), a rotation of the lever element (19) by a quarter rotation is required.

6. Arrangement (1) as claimed in any one of the preceding claims, wherein at least one switching process from the second switching position (17) into the first switching position (16) is effectuatable automatically.

7. Arrangement (1) as claimed in any one of the preceding claims, wherein the switchable connector (2) and the gas flow reversing element (3) are detachably connectable to one another.

8. Arrangement (1) as claimed in any one of claims 1 to 6, wherein the switchable connector (2) and the gas flow reversing element (3) are connected to one another in a non-detachable manner.

9. Arrangement (1) as claimed in any one of the preceding claims, wherein at least one connection (20) of the group switchable connector (2) with line connector (6), switchable connector (2) with second line (15), and switchable connector (2) with first line (13) is embodied as a so-called "Luer lock" connection.

## Revendications

1. Agencement (1), comprenant un raccord commutable (2) et un élément d'inversion de flux de gaz (3), qui est approprié pour utiliser une réserve de gaz (4) soumise à une surpression pour générer de manière sélective un flux de gaz (5) depuis ou vers un raccord de conduite (6), qui peut être connecté par le biais d'une lumière (7) de l'agencement (1) à une voie respiratoire d'un patient (8), l'élément d'inversion de flux de gaz (3) étant réalisé au moins sous forme de pièce principale (9) avec une pièce de dérivation (10), la pièce principale (9) reliant un raccord de pression (11) pour le raccordement à la réserve de gaz (4) à au moins une ouverture de sortie refermable (12), et la pièce de dérivation (10) reliant par une première conduite (13) la pièce principale (9) au raccord de conduite (6), une buse (14) étant réalisée et disposée dans la pièce principale (9) de telle sorte qu'à partir d'un flux de gaz (5) dans la pièce principale (9) s'écoulant depuis le raccord de pression (11) à travers la buse (14) jusqu'à l'ouverture de sortie (12) lorsque l'ouverture de sortie (12) est ouverte, un flux de gaz (5) puisse également être généré à travers la première conduite (13) dans la direction de l'ouverture de sortie (12), le raccord commutable (2) étant disposé au niveau de la pièce de dérivation (10) ou au niveau du raccord de conduite (6) et reliant de manière commutable au moins une deuxième conduite (15) de l'agencement (1) à la première conduite (13), le raccord commutable (2) présentant au moins deux positions de commutation (16, 17), dans lequel
1) dans la première position de commutation (16), la première conduite (13) est ouverte et la deuxième conduite (15) est fermée, de telle sorte que le flux de gaz (5) puisse s'écouler à travers le raccord commutable (2) dans les deux directions le long de la première conduite (13) ;
2) dans la deuxième position de commutation (17), si le raccord commutable (2) est disposé au niveau de la pièce de dérivation (10), la deuxième conduite (15) est connectée fluidiquement par le biais du raccord commutable (2) au raccord de conduite (6) et à la lumière (7), par le biais de laquelle le raccord de conduite (6) peut être connecté à la voie respiratoire du patient, ou, si le raccord commutable (2) est disposé au niveau du raccord de conduite (6), la deuxième conduite (15) est connectée fluidiquement à la lumière (7), par le biais de laquelle le raccord de conduite (6) peut être connecté à la voie respiratoire du patient ; et
dans la deuxième position de commutation (17), la première conduite (13) est fermée vers la pièce principale (9) et la deuxième conduite (15) peut être connectée fluidiquement par le biais du raccord commutable (2) seulement au raccord de conduite et/ou à la lumière (7), par le biais de laquelle le raccord de conduite (6) peut être connecté à la voie respiratoire du patient ; et
de telle sorte que la deuxième conduite (15) ne puisse à aucun instant être connectée fluidiquement à la pièce principale (9).

2. Agencement (1) selon la revendication 1, dans lequel l'élément d'inversion de flux de gaz (3) est disposé dans un boîtier (18) de forme ergonomique, qui est adapté à la main d'une personne, le raccord commutable (2) pouvant être transféré manuellement, de préférence par au moins un doigt de la même main d'une personne, au moins de la première position de commutation (16) à la deuxième position de commutation (17).

3. Agencement (1) selon l'une quelconque des revendications précédentes, dans lequel le raccord (2) peut être actionné par le biais d'un élément de levier (19).

4. Agencement (1) selon la revendication 3, dans lequel l'élément de levier (19) est disposé de manière rotative sur le raccord (2) pour l'actionnement du raccord (2).

5. Agencement (1) selon la revendication 4, dans lequel, pour une opération de commutation entre la première position de commutation (16) et la deuxième position de commutation (17), une rotation de l'élément de levier (19) d'un quart de tour est nécessaire.

6. Agencement (1) selon l'une quelconque des revendications précédentes, dans lequel au moins une opération de commutation de la deuxième position de commutation (17) à la première position de commutation (16) peut être effectuée automatiquement.

7. Agencement (1) selon l'une quelconque des revendications précédentes, dans lequel le raccord commutable (2) et l'élément d'inversion de flux de gaz (3) peuvent être connectés l'un à l'autre de manière amovible.

8. Agencement (1) selon l'une quelconque des revendications 1 à 6, dans lequel le raccord commutable (2) et l'élément d'inversion de flux de gaz (3) sont connectés l'un à l'autre de manière non amovible.

9. Agencement (1) selon l'une quelconque des revendications précédentes, dans lequel au moins une connexion (20) dans le groupe comprenant le raccord commutable (2) au raccord de conduite (6), le raccord commutable (2) à la deuxième conduite (15) et le raccord commutable (2) à la première conduite (13) est réalisé sous la forme de ce qu'on appelle un "raccord de Luer".
